# EUROPEAN PATENT APPLICATION

(11) **EP 4 166 122 A2**
(43) Date of publication of application: **19.04.2023**
(21) Application number: 22174071.5
(22) Date of filing: 17.06.2019
(51) Int. Cl.: A61F 13/38, D21C 9/00, D01G 13/00, D01G 15/00, D04H 1/02, D04H 1/26

(54) **SOLUBLE ARTICLES AND MANUFACTURE AND DISPOSAL THEREOF**

(30) Priority: 15.06.2018 GB 201809891; 15.06.2018 GB 201809894; 15.06.2018 GB 201809895; 15.06.2018 GB 201809896
(62) Divisional of application: 19744780.8
(71) Applicant: Pellis Innovations Limited, Edinburgh EH3 8PB (GB)
(72) Inventor: McCormack, Brian James, Fife, KY2 6QS (GB)
(74) Representative: Symbiosis IP Limited

(57) **Abstract**

A method for making an absorbent cotton wool type material such as a cotton bud; an absorbent pad which comprises an outer water impermeable layer and the inner water permeable layer between which there is contained soluble fibres; and a sanitary macerator comprising a chamber which is divided into two parts, a first part contains an area in which the material to be macerated is placed which is closed by a cover and a second part which contains a macerator.

## Description

A first aspect of the present invention relates to a dissolvable cotton bud.

Cotton buds are regularly used absorbent material. The material used for the bud in these cotton buds today is synthetic fibres such as polyester or nylon and the shaft of the cotton bud is made from plastics material. Such cotton buds are widely used for sanitary, medical and cosmetic purposes. The disposal of these cotton buds creates significant environmental issues. Whilst medical waste is often incinerated, most of the cotton buds either ends up in landfill or flushed down the sewage system. This creates considerable blockage problems in any sewage system. These cotton buds also end up in ocean and create extremely persistent problems as recently highlighted in the UK Press.

A first aspect of the present invention seeks to address this problem.

Accordingly a first aspect of the present invention is directed to a cotton bud, in which a shaft is made from rolled dissolvable paper and the buds at the ends comprise entangled dissolvable cotton wool type fibres.

This provides the advantage that the whole of the cotton bud after use can be dissolved in a short time frame in water. Therefore should the cotton bud be disposed of down a sewage system providing a reasonable flow of water occurs the cotton bud will dissolve completely. Furthermore should such a cotton bud find its way into the ocean similarly the cotton bud will dissolve.

The entangled dissolvable paper fibres with cotton wool like properties are described below. This provides the advantage that the fibres are both absorbent, have a reasonable use period and dissolve in their entirety.

Advantageously the hollow shaft contains compressed entangled such paper fibres which can be pulled out when the existing bud needs to be refreshed after used. In a preferred embodiment the compressed fibres have pre-scored breaks in order to break off the used part once the fresh part has been pulled out. This provides the advantage of providing further life to the cotton bud.

In a preferred embodiment the dissolvable paper of the shaft is carboxy methyl cellulose. Advantageously it is coated with an appropriate soluble coating in order to provide added stiffness and endurance. Alternatively the rolled paper can be a multilayer paper held together by dissolvable glue. This provides the advantage of additional durability of the shaft.

A second and third aspect of the present invention relates to an absorbent paper material.

Cotton wool or cotton pads are regularly used absorbent material. While sometimes sold as balls they are longer made from natural cotton fibres. This is largely caused by the United States Federal Trade Commission, which has stringent requirements for the labelling of textile products. The material used for the cotton wool today is synthetic fibres such as polyester or nylon. Such cotton wool/pads and products containing them are widely used for sanitary, medical and cosmetic purposes. The disposal of this material creates significant environmental issues. Whilst medical waste is often incinerated, most of the other material either ends up in landfill or flushed down the sewage system. This creates a considerable blockage problems in any sewage system. Cotton wool is also extremely persistent in land fill.

A second and third aspect of the present invention seeks to address this problem.

Accordingly a second aspect of the present invention is directed to a method of making an absorbent cotton wool type material comprising the steps of: a) creating a blend of water soluble fibres; b) subjecting the fibres to rotational motion in a confined chamber; and c) carding the fibre conglomerations created to disentangle and intermix the fibres.

This provides the advantage of a simple method to produce a soluble fibre product with qualities similar to cotton wool.

Advantageously the soluble fibre is comprised of sodium carboxyl methyl cellulose, wood pulp or combinations thereof. This has the advantage that these fibres are readily available and will dissolve in water in a reasonable time. In a preferred embodiment the starting material is 10 point (252 gsm) Facestock calendered water soluble paper.

In a preferred embodiment the confined chamber is loosely packed with the soluble fibres to allow aeration of the fibres on mixing. Advantageously further soluble material can be added to the soluble fibres to create the desired material properties.

Advantageously rotational blending with high speed cutting blades occurs for approximately 1 minute, which is repeated a number of times to achieve the required result. These times can be varied to achieve the product characteristics required.

In a preferred embodiment in the carding of the conglomerations to create the final product, the fibres can be mixed with in other fibres in order to achieve longevity for the absorbent material when exposed to fluid or alter the material properties.

Accordingly a third aspect of the invention is directed to an absorbent fibre material made according to the method described above. This provides a product which has the absorbent qualities of cotton wool whilst allowing for on continued exposure to water complete dissolution. This means that in future sewage systems will no longer be blocked by cotton wool and in landfill such products will break breakdown. In simple terms the product could be referred to as cotton wool type material.

The cotton wool type material provides a replacement for cotton wool in all the products made using cotton wool.

A fourth aspect of the present invention relates to a dissolvable absorbent pads.

Absorbent pads include sanitary towels, nappies/diapers, incontinence pads and mattress protectors. The material used for to absorb in these pads is synthetic fibres and super absorbent fabrics. Often they also contain sheets of plastics materials as impermeable outer covers or one way moisture barriers. Such pads are widely used for sanitary, medical and cosmetic purposes. The disposal of these pads creates significant environmental issues. Whilst medical waste is often incinerated, most pads either ends up in landfill or flushed down the sewage system. This creates considerable blockage problems in any sewage system. These pads also end up in ocean and create extremely persistent problems as recently highlighted in the UK Press.

A fourth aspect of the present invention seeks to address this problem.

Accordingly a fourth aspect of the present invention is directed to an absorbent pad which comprises an outer water impermeable layer and the inner water permeable layer between which there is contained soluble fibres.

This provides the advantage that the absorbent pad is dissolvable.

Advantageously the soluble fibre is comprised of sodium carboxyl methyl cellulose, wood pulp or combinations thereof. This has the advantage that these fibres are readily available and will dissolve in water in a reasonable time. In a preferred embodiment the starting material is 10 point (252 gsm) Facestock calendered water soluble paper.

The soluble fibres are advantageously entangled dissolvable fibres ("cotton wool type material") described above. This provides the advantage that the fibres are both absorbent, have a reasonable use period and dissolve in their entirety. The absorbent properties are improved by the addition of polyacrylate either by mixing or as a layer.

In a preferred embodiment the inner and outer layers are made from water soluble material preferably water-soluble plastics material or soluble fabric. This provides the advantage that the pad in totality is dissolvable and therefore will not create problems in any sewage system and is especially suitable for use as a mattress protector.

Preferably the outer and inner layer comprises of a direct material that dissolves or disperses above 60°C in water. This provides the advantage that the pad remains solid until such time as it is decided to dispose of it, which can be done using hot water. Alternatively the outer layer comprises direct thermal soluble paper with an integrated water proof membrane. This is particularly advantageous with regard to hospital waste where such waste will need to be decontaminated by autoclaving etc.

In a preferred embodiment the pad includes anti-bacterial substances. These substances can either be added as additional material or impregnated into the existing material or into one of the layers. This provides added protection for users of such pads from infection when they are used over a long period of time.

The intended outer layer of the pad can comprise soluble fabric and can additionally be coated with adhesive to hold it to other external items. This is advantageous in the instance of panty liners.

In a preferred embodiment the pad is one of the following sanitary towel, nappy, diaper, incontinence pads and mattress protectors.

This provide the advantage these comprise a wide variety of items that are in regular use for which the disposal creates significant environmental issues. Thus the use of the present invention in these applications provides significant advantage.

Advantageously the pads are equipped with the necessary additional features for the relevant application to make them work such as adhesive strips in the case of nappies, incontinence pads and sanitary towels. The adhesive will be selected from one that is water-soluble and additional straps added to the outer layer to achieve attachment will again be of the same material as the outer layer. Allowing for easy welding or adhesion but with the necessary dissolution.

A fifth aspect of the present invention relates to a sanitary macerator.

Sanitary macerators are well-known equipment for dealing with human waste from a toilet and other body fluids from treatment of patients that require disposal by maceration. In a hospital there are often problems in that such macerators are expected to deal with the products of bedpans and soiled articles such as bedcovers. Often the bedpans will have additional lining material and similarly disposable bed mattress protectors, the type used for toileting of bed ridden patients, can be of a variety of materials some of which may not be hygienically disposed through a macerator.

It is an aim of the fifth aspect of the present invention to provide a sanitary macerator which addresses these problems.

Accordingly a fifth aspect of the present invention is directed to a sanitary macerator comprising a chamber which is divided into two parts, a first part contains an area in which the material to be macerated is placed which is closed by a cover and a second part which contains a macerator. The material in the chamber is heated before any maceration starts by hot water at a predetermined temperature preferably at least 60°C which is released onto the material to be macerated. Materials to be used in this macerator are preferably those that dissolve at about 60°C.

On use the chamber is closed by the cover, which allows operation of the macerator, the macerator has an inlet which lets hot water into the first part of the chamber and an outlet which removes the macerated waste from the second part. The inlet has a valve that allows in water to the macerator with a temperature above a predetermined level. The macerating process runs for 30 seconds and then the contents can be flushed away with cold water.

This provides the advantage that when a bedpan liner or a bed mattress protector is placed in the first part of the chamber with human waste/body fluids the heated water is allowed in which dissolves the bedpan liner or a bed mattress protector which is made of a material that dissolves above the predetermined temperature. This allows the bed mattress protector or bedpan liner to dissolve thus allowing the macerator to deal with the waste in a normal fashion without becoming clogged by any bedpan liner or sheet material which are present as pulp based material.

In a preferred embodiment the water inlet has a heater included with it to ensure the water supplied is to a predetermined temperature. Preferably the first part is above the second part allowing gravity to move the waste through the macerator. Advantageously the macerator has a control device that operates the macerator once the contents of the first part has been exposed to heated water for a predetermined period of time. This provides the advantage that upon an operator placing the material to be processed in the macerator it will proceed with operation in a timely fashion without further attention. In a preferred embodiment once the contents of the macerator has been cleared further water is allowed in though the valve to clean and flush the macerator.

Preferably an operating control operates the various parts of this process to maximum efficiency and there may be a sensor to check if the soiled articles have dissolved before maceration starts.

The invention also extends to a method of operating a macerator in accordance with a fifth aspect of the present invention.

Examples made in accordance with the present invention will now be described hereinbelow with reference to the attached drawings, in which:
Figure 1 is a perspective view of a cotton bud according to a first aspect of the present invention;
Figure 2 is a cross-sectional view along Plane A of the cotton bud in Figure 1;
Figure 3 is a cross-sectional view of the cotton bud in Figure 1 along plane B;
Figure 4 is a sectional view of a pad according to a fourth aspect of the present invention;
Figure 5 is a top view of a sanitary towel/panty liner according to a fourth aspect of the present invention;
Figure 6 is a top view of a nappy/diaper/incontinence pad according to a fourth aspect of the present invention;
Figure 7 is a top view of a mattress protector according to a fourth aspect of the present invention; and
Figure 8 shows a side sectional view of a macerator according to a fifth aspect of the present invention.

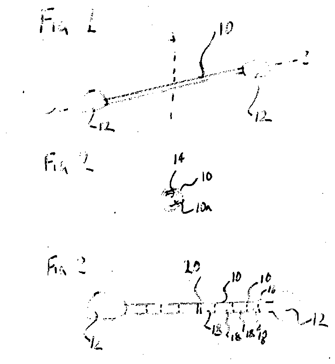

Figure 1 shows a perspective view of a cotton bud comprising a shaft 10 with buds 12 at either end. The buds 12 are secured to the shaft 10 either by external glue or alternatively through attachment to compressed fibre in the shaft 10. The cotton buds 12 are made from entangled dissolvable paper fibres with cotton wool like properties. The shaft 10 is made from rolled dissolvable paper. The fibres of the buds 12 and the paper of the shaft 10 is made preferably from carboxy methyl cellulose and/or wood fibre.

Figure 2 shows a cross-section along plane A in Figure 1 through the shaft 10 of the cotton bud. The shaft 10 comprises rolled dissolvable paper which may have multiple layers with a width of 10a, which are glued together by a water soluble glue. The exterior of the shaft 10 may be coated with a suitable dissolving material to provide extra strength and rigidity. The centre 14 of the shaft 10 is hollow. This can be filled with compressed entangled dissolvable fibres.

Figure 3 shows a cross-sectional view along plane B in Figure 1 of the cotton bud showing the shaft 10 containing a series of compressed buds 18 which are separated by pre-scorings 16 to enable these to be broken when a fresh bud 12 upon use is pulled out of the centre 14 of the shaft 10. There is a break 20 in the middle as both halves of the shaft 10 contain compressed entangled fibre material for the buds 12 at each end.

In use the cotton bud, once the bud 12 becomes soiled can have the bud 12 pulled off pulling out of the shaft 10 a further bud 12. The new bud 12 is separated from the old bud 12 by the pre-scoring 18. Owing to the dissolvable nature of the bud 12, this can easily be disposed of by washing down a sink or a lavatory with no likelihood of blockage.

In an example according to a second and third aspect of the present invention raw material/feedstock namely 10pt (252gsm) Facestock (calendered water soluble paper) comprised of sodium carboxyl methyl cellulose and wood pulp was provided. The feedstock was placed in a 400W food blender with a gearless motor. The packing of the fibre material into the blender was loose to facilitate aeration. The feedstock was blended for 1 minute and allowed to settle. This was repeated twice to produce the required consistency of the product. The product is comprised of loose blended cotton wool type material.

A preferred embodiment of the object is to form 1 inch balls of cotton wool type material, i.e. a cotton wool type ball, which weigh half an ounce.

A cotton wool type ball produced by this method was able to mop up small water spillages without losing form. Once the ball was placed in a glass of water dissolution occurred within 1 minute and with slight agitation complete dissolution was achieved, leaving no large pieces of fibre material.

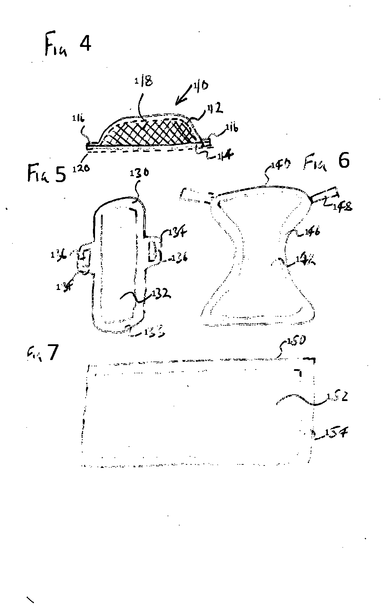

Figure 4 shows a cross-section of a dissolvable absorbent pad 110 comprising an inner layer 112 and an outer layer 114. The inner layer 112 is joined to the outer layer 114 at edge 116. This join 116 can be through adhesion or welding and goes for all the way round the pad 110. The pad 110 may have multiple planar geometries depending in its intended use. Between the inner layer 112 and the outer layer 114 is held soluble paper fibres 18. The soluble paper fibres 118 are preferably entangled and held together as a mesh. The fibres 118 may contain additional elements treated with antimicrobial compounds alternatively inner layer 112 or outer layer 114 may also be treated.

The outer layer 114 is made water impermeable by the addition of a layer 120 and is intended to hold water absorbed by the soluble fibres 118 though the inner layer 112 from reaching the outside of the pad 110. The inner layer 112 is advantageously semi-permeable from the outside to the inside. This enables water or moisture to be absorbed through the inner layer 112 and held by the soluble fibres 118.

The outer and inner layers 112, 114 can be made of soluble material such that when the pad 110 is placed in water it dissolves as a whole. This leads to no likelihood of the blocking drains and easy dissolution if placed in landfill. In the case of use in hospital ideally these layers 112, 114 dissolve at a higher temperature. This means that while in general use they will keep absorb fluids such as blood, urine or other bodily fluids intact and are only dissolved when placed in the appropriate cleansing apparatus for disposal.

The products shown in Figures 5 to 7 all have the sectional structure shown in Figure 4.

Figure 5 shows a plan view of a sanitary towel/panty liner 130. The sanitary towel 130 has the structure described in Figure 4 with the soluble fibres contained in the central part 132 of the sanitary towel 130 and welded edge 133 matches the desired contour of the product. Wings 134 are provided on the sanitary towel 130 for the attachment of adhesive portions 136 for use. The outer layer 114 in this case can be coated with an adhesive to hold in on the pants.

Figure 6 shows a nappy or incontinence pack 140. The nappy 140 again has the structure described in Figure 4 with the soluble fibres contained in the central part 132 of the nappy 140 and welded round the edge 146 which matches the desired contour of the product. The nappy 140 is equipped with soluble adhesive tabs 48 for fitting to the baby or adult when in use.

Figure 7 shows a plan view of a bed sheet 150 which again has the structure described in Figure 4 with the soluble fibres contained in the central part 152 of the sheet 150 and welded round the edge 154 which matches the desired contour of the product. The welded outer edge 154 can be larger in this case or have added features such as elastic edges to fit on a bed.

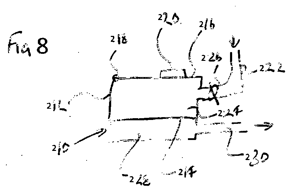

Figure 8 shows a side view of a macerator 210 according to a fifth aspect of the present invention with a chamber 212 divided into two parts by separator 214. The top part of the chamber has a door 216 hinged at one corner 218 to enable the door 216 to be lifted up by handle 218. A pipe 222 supplying hot water flows into the top part 224 through valve 226. The macerator 228 is housed in the lower part 228 of the chamber 212. The macerated product is expelled through pipe 230 into the sewage system.

In operation a handle 220 is lifted up and the soiled article comprising a bed liner or suchlike that dissolves at a higher temperature, preferably 60°C, is placed in chamber 212. The valve 226 is opened and hot water from pipe 222 flows into the chamber 212. After a predetermined time providing the door 216 is closed the macerator 228 is operated and the resultant sewage is expelled through pipe 230. Further water then flows in through valve 226 to assist with flushing when required and to clean the macerator. Ideally a control operates the various parts of this process to maximum efficiency and there may be a sensor to check if the soiled articles have dissolved before maceration starts.

## Claims

1. A method of making an absorbent cotton wool type material comprising the steps of: a) creating a blend of water soluble fibres; b) subjecting the fibres to rotational motion in a confined chamber; and c) carding the fibre conglomerations created to disentangle and intermix the fibres.

2. A method according to Claim 1, in which the soluble fibre is comprised of sodium carboxyl methyl cellulose, wood pulp or combinations thereof.

3. A method according to Claim 1or Claim 2, in which the starting material is 10 point (252 gsm) Facestock calendered water soluble paper.

4. A method according to any one of Claims 1 to 3, in which the confined chamber is loosely packed with the soluble fibres to allow aeration of the fibres on mixing.

5. A method according to any one of Claims 1 to 4, in which further soluble material can be added to the soluble fibres to create the desired material properties.

6. A method according to any one of Claims 1 to 5, in which ro- tational blending with high speed cutting blades occurs for ap- proximately 1 minute which is repeated a number of times to achieve the required result.

7. A method according to any one of Claims 1 to 6, in which the carding of the conglomerations to create the final product, the fibres can be mixed with in other fibres in order to achieve longevity for the absorbent material when exposed to fluid or alter the material properties.

8. An absorbent fibre material made according to the method in any one of Claims 1 to 7.

9. An absorbent pad which comprises an outer water impermeable layer and the inner water permeable layer between which there is contained soluble fibres.

10. An absorbent pad according to Claim 9, in which the soluble fibre is comprised of sodium carboxyl methyl cellulose, wood pulp or combinations thereof.

11. An absorbent pad according to Claim 9 or Claim 10, in which the starting material is 10 point (252 gsm) Facestock calendered water soluble paper.

12. An absorbent pad according to any one of Claims 9 to 11, in which the soluble fibres are advantageously entangled dis- solvable fibres.

13. An absorbent pad according to any one of Claims 9 to 12, in which the absorbent properties are improved by the addition of polyacrylate either by mixing or as a layer.

14. An absorbent pad according to any one of Claims 9 to 13, in which the inner and outer layers are made from water soluble material preferably water-soluble plastics material or soluble fabric.

15. An absorbent pad according to any one of Claims 9 to 14, in which the outer and inner layer comprises of a direct material that dissolves or disperses above 60°C in water.

16. An absorbent pad according to any one of Claims 9 to 15, in which the outer layer comprises direct thermal soluble paper with an integrated water proof membrane.

17. An absorbent pad according to any one of Claims 9 to 16, in which the pad includes anti-bacterial substances.

18. An absorbent pad according to any one of Claims 9 to 17, in which the intended outer layer of the pad comprises soluble fabric and/or is additionally be coated with adhesive to hold it to other external items.

19. An absorbent pad according to any one of Claims 9 to 18, in which the pad is one of the following sanitary towel, nappy, diaper, incontinence pads and mattress protectors.

20. An absorbent pad according to any one of Claims 9 to 19, in which the pads are equipped with the necessary additional features for the relevant application to make them work such as adhesive strips in the case of nappies, incontinence pads and sanitary towels.

21. An absorbent pad according to Claim 20, in which the adhesive is selected from one that is water-soluble and additional straps added to the outer layer to achieve attachment will again be of the same material as the outer layer.

22. A sanitary macerator comprising a chamber which is divided into two parts, a first part contains an area in which the material to be macerated is placed which is closed by a cover and a second part which contains a macerator.

23. A sanitary macerator according to Claim 22, in which closure of the cover allows operation of the macerator.

24. A sanitary macerator according to Claim 22 or Claim 23, in which the macerator has an inlet which lets hot water into the first part of the chamber and an outlet which removes the macerated waste from the second part.

25. A sanitary macerator according to any one of Claims 22 to 24, in which the inlet has a valve that allows in water to the macerator with a temperature above a predetermined level.

26. A sanitary macerator according to any one of Claims 22 to 25, in which the water inlet has a heater included with it to ensure the water supplied is to a predetermined temperature.

27. A sanitary macerator according to any one of Claims 22 to 26, in which the first part is above the second part allowing gravity to move the waste through the macerator.

28. A sanitary macerator according to any one of Claims 22 to 27, in which the macerator has a control device that operates the macerator once the contents of the first part has been exposed to heated water for a predetermined period of time.

29. A sanitary macerator according to any one of Claims 22 to 28, in which once the contents of the macerator has been cleared further water is allowed in though the valve to clean and flush the macerator.

30. A sanitary macerator according to any one of Claims 22 to 29, in which an operating control operates the various parts of the process to maximum efficiency and a sensor is provided to check if the soiled articles have dissolved before maceration starts.
